# DEMANDE DE BREVET EUROPEEN

(11) **EP 2 952 174 A1**
(43) Date de publication de la demande: **09.12.2015**
(21) Numéro de dépôt: 15170034.1
(22) Date de dépôt: 01.06.2015
(51) Int. Cl.: A61K 8/67, A61K 8/68, A61Q 19/00, A61K 8/97

(54) **COMPOSITIONS DE SOINS DE LA PEAU**

(30) Priorité: 03.06.2014 FR 1455033
(71) Demandeur: CASTER, 75008 Paris (FR)
(72) Inventeur: Choulot, Jean-Christophe, 78120 RAMBOUILLET (FR); Hajem, Neïla, 78290 CROISSY SUR SEINE (FR)
(74) Mandataire: Casalonga

(57) **Abrégé**

La présente invention a pour objet une composition contenant au moins du phosphate d'ascorbyle de magnésium, un extrait de pois, un extrait de crocus et un composé céramide de formule (I). L'invention a également pour objet l'utilisation d'une telle composition pour améliorer l'effet barrière, l'élasticité et la fermeté de la peau ainsi que pour densifier le derme et stimuler la synthèse de collagène de la peau.

## Description

La présente invention a pour objet une composition contenant au moins du phosphate d'ascorbyle de magnésium, un extrait de pois, un extrait de crocus et un composé de formule (I). L'invention a également pour objet l'utilisation d'une telle composition pour améliorer l'effet barrière, l'élasticité et la fermeté de la peau ainsi que pour densifier le derme et stimuler la synthèse de collagène de la peau.

La peau est l'organe de revêtement recouvrant la totalité de la surface du corps, constituée de trois compartiments distincts superposés : l'épiderme, le derme et l'hypoderme.

L'épiderme est un épithélium squameux stratifié qui constitue la structure externe de la peau et assure sa fonction de protection. Les kératinocytes sont le principal type cellulaire représenté dans l'épiderme. L'épiderme n'est irrigué par aucun vaisseau sanguin. Il contient par contre de nombreuses terminaisons nerveuses libres, permettant d'avoir une perception tactile et douloureuse. Au sein de l'épiderme, quatre couches sont à distinguer :
- la couche cornée *(Stratum corneum)* est une couche constituée de cellules très épaisses, les cornéocytes qui ne possèdent pas de noyau.
- la couche granuleuse *(Stratum granulosum)* est une couche contenant 3 ou 4 assises de cellules.
- la couche des cellules à épines *(Stratum spinosum*) contient des kératinocytes, des tonofilaments précurseurs de la kératine, des mélanocytes et des terminaisons nerveuses.
- la couche basale *(Stratum germinativum*) où les kératinocytes forment une seule assise de cellules, tenues entre elles par des desmosomes. C'est dans cette couche dite aussi couche germinative, que les cellules se divisent.

Le derme est un tissu de soutien. Il se compose de protéines fibreuses dont le collagène (de types I et III en particulier) et l'élastine, qui participent à la résistance, l'élasticité et surtout à la tonicité de la peau et/ou des muqueuses. Le derme est irrigué par un réseau sanguin microcirculatoire qui prend en charge la nutrition de l'épiderme par diffusion. Outre son rôle nutritif, le derme joue également un rôle primordial dans la thermorégulation chez l'homme et dans la cicatrisation ainsi que dans l'élimination de produits toxiques par la sueur.

Au-dessous du derme se trouve un tissu conjonctif lâche richement vascularisé : l'hypoderme.

L'épiderme, épithélium de revêtement a pour fonction particulière de constituer une barrière protégeant efficacement le milieu interne de l'environnement et, en tout premier lieu, de la dessication. En effet, la barrière cutanée protège l'organisme contre des facteurs externes de contraintes physiques (facteurs mécaniques, thermiques et rayons UV, notamment), chimiques (tensioactifs, exposition prolongée à l'eau, solvants, etc.) et environnementaux. De plus, la peau, en tant que barrière, protège l'organisme contre la perte d'ions essentiels, d'eau et de protéines sériques.

La fonction primaire de l'épiderme est ainsi de produire la couche cornée qui forme une couche protectrice semi-perméable. L'épaisseur moyenne de l'épiderme est de 100 µm mais peut varier de 50µm sur les paupières à 1 mm sur la paume des mains ou la plante des pieds.

Le système nerveux cutané constitue une partie du système nerveux périphérique. Le système nerveux cutané est riche et complexe avec une voie afférente et une voie efférente, les trois compartiments cutanés, hypoderme, derme, et épiderme (sauf la couche cornée) étant innervés.

La peau étant le principal site d'interaction de l'organisme avec son environnement, le système nerveux cutané reçoit et répond continuellement à toute une variété de stimuli qui peuvent être physiques (thermique, mécanique, électrique, rayonnement UV), chimiques, ou indirects tels que ceux produits par des allergènes, des haptènes, des agents microbiologiques, des chocs ou une inflammation. Les nerfs cutanés peuvent également répondre à des stimuli issus de la circulation sanguine ou à des stress émotionnels.

La densité d'innervation varie en fonction de la zone du corps, ainsi, chez les humains, les mains et le visage sont richement innervés par rapport au dos ou à l'abdomen.

Des contacts étroits entre fibres nerveuses et cellules cutanées ont été observés. Les fibres nerveuses de la peau produisent des neuromédiateurs (dont le calcitonin-gene related peptide (CGRP) et des neurohormones. Le CGRP est un des principaux médiateurs de la peau et stimule en particulier la prolifération et la migration cellulaires (Chéret. J et al., J Dermatol Sci. 2014 Jun;74(3):193-203. Epub 2014 Fev. 16).

En réponse, les cellules de l'épiderme libèrent des cytokines et des facteurs de croissance.

Il existe donc un dialogue entre les cellules de la peau et les neurones sensitifs. De plus, le système nerveux central peut moduler soit directement par les nerfs efférents ou des médiateurs chimiques, ou indirectement, un grand nombre de fonctions au sein de la peau, dont la vasomotricité, la thermorégulation, la sécrétion des glandes et des cellules, la croissance et la différenciation des tissus, ou encore la fonction barrière de la peau.

Il subsiste cependant un besoin de compositions permettant d'améliorer la fonction barrière de la peau face aux agressions journalières qu'elle subit, tout en maintenant et améliorant son élasticité, sa fermeté et sa densité.

La demanderesse a ainsi mis au point de nouvelles compositions topiques de soins de la peau, qui constituent l'objet de l'invention.

L'invention a également pour objet un procédé de soin pour améliorer l'effet barrière, l'élasticité et la fermeté de la peau ainsi que pour densifier le derme et stimuler la synthèse de collagène de la peau, comprenant l'application sur la peau d'une composition telle que définie précédemment.

La présente invention a également pour objet l'utilisation de la composition selon l'invention pour améliorer l'effet barrière, l'élasticité et la fermeté de la peau.

La présente invention a également pour objet l'utilisation de la composition selon l'invention pour densifier le derme et stimuler la synthèse de collagène de la peau.

D'autres objets, caractéristiques, aspects et avantages de l'invention apparaîtront encore plus clairement à la lecture de la description et des exemples qui suivent.
La figure 1 montre l'effet des compositions S1 à S3 à différentes dilutions, allant de 0.1x à 0.04x pour chaque composition, sur la survie des neurones sensitifs. Les donnés sont exprimées en pourcentage par rapport à la moyenne du témoin, 100% étant considéré comme le milieu contrôle).
La Figure 2 montre l'effet des compositions S1 et S2 à 0.02x et 0.04x sur la libération de CGRP par les neurones sensitifs. Les données sont exprimées en pourcentage par rapport à la moyenne du témoin +/- SEM (100 % = milieu contrôle). *p<0.05, **p<0.01, ***p<0.005
La figure 3 montre la morphologie d'un explant du lot témoin plastie à J-1
La figure 4 montre la morphologie d'un explant du lot témoin à J0, (T0)
La figure 5 montre la morphologie d'un explant du lot non traité à 8h, (T8h)
La figure 6 montre la morphologie d'un explant du lot traité avec P à 8h, (P8h)
La figure 7 montre la morphologie d'un explant du lot non traité à 24h, (T24h)
La figure 8 montre la morphologie d'un explant du lot traité avec P à 24h, (P24h)
La figure 9 montre la morphologie d'un explant du lot non traité à J9, (TJ9)
La figure 10 montre la morphologie d'un explant du lot traité avec P à J9, (PJ9)

Les compositions conformes à l'invention comportent dans un milieu physiologiquement acceptable, au moins du phosphate d'ascorbyle de magnésium, un extrait de pois, un extrait de crocus et un composé céramide de formule (I).

Le phosphate d'ascorbyle de magnésium est de préférence dissous dans du glycérol et de l'éthanol.

De préférence, le phosphate d'ascorbyle de magnésium est présent dans des proportions de 0.6 à 5 % en poids par rapport au poids total de la composition, et encore plus préférentiellement dans des proportions de 0.1 à 10% en poids par rapport au poids total de la composition.

Comme source de phosphate d'ascorbyle, on pourra utiliser la dispersion de liposomes vendue sous le nom de Lipoid par la société Cosmetochem (Suisse).

La composition conforme à l'invention comprend également un extrait de pois, et plus particulièrement un extrait du pois *Pisum sativum*.

De préférence, l'extrait de *Pisum sativum* est présent dans des proportions de 0.2 à 10% en poids et encore plus préférentiellement dans des proportions de 0.5 à 5% en poids par rapport au poids total de la composition.

Comme source d'extrait de *Pisum sativum*, on pourra utiliser la solution liquide contenant 3% de matière active de la société BASF, vendue sous le nom de Proteasyl TP POE LS 9818.

La composition selon l'invention comprend également un extrait de bulbe de crocus, et plus particulièrement un extrait de bulbe de *Crocus chrysanthus.*

De préférence, l'extrait de bulbe de *Crocus chrysanthus* est présent dans des proportions de 0.2 à 10% en poids et encore plus préférentiellement dans des proportions de 0.5 à 5% en poids par rapport au poids total de la composition.

Comme source d'extrait de bulbe de *Crocus chrysanthus,* on pourra utiliser l'extrait de bulbe de Crocus chrysanthus à 1% de la société Mibelle, vendue sous le nom de DermCom.

La composition selon l'invention comprend également un composé céramide de formule (I)

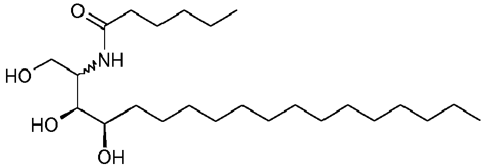

De préférence, le composé de formule (I) est présent dans des proportions de 0.1 à 5% en poids et plus préférentiellement de 0.2 à 3% en poids par rapport au poids total de la composition.

Comme source de composé céramide de formule (I), on peut utiliser la sphingokine NP vendue par la société Evonik.

La composition selon l'invention est adaptée à une application topique sur la peau et comprend donc généralement un milieu physiologiquement acceptable, c'est-à-dire compatible avec la peau. Il s'agit de préférence d'un milieu cosmétiquement acceptable, c'est-à-dire qui présente une couleur, une odeur et un toucher agréables et qui ne génère pas d'inconforts inacceptables (picotements, tiraillements, rougeurs), susceptibles de détourner la consommatrice d'utiliser cette composition.

La composition selon l'invention peut comprendre avantageusement une phase aqueuse. La composition peut comprendre de l'eau en une teneur allant de 10% à 90% en poids par rapport au poids total de la composition, de préférence allant de 50% à 70% en poids par rapport au poids total de la composition.

La composition selon l'invention peut se présenter sous toutes les formes galéniques normalement utilisées pour une application topique sur la peau, notamment sous forme d'une émulsion huile-dans-eau ou eau-dans-huile ou multiple, éventuellement gélifiée, d'une émulsion siliconée, d'une microémulsion ou nanoémulsion, d'un produit anhydre liquide, pâteux ou solide, d'une dispersion d'huile dans une phase aqueuse en présence de sphérules, ces sphérules pouvant être des nanoparticules polymériques telles que les nanosphères et les nanocapsules ou, mieux, des vésicules lipidiques de type ionique et/ou non-ionique.

Cette composition peut être plus ou moins fluide et avoir l'aspect d'une crème blanche ou colorée, d'une pommade, d'un lait, d'une lotion, d'un sérum, d'une pâte, d'une mousse ou d'un gel. Elle peut éventuellement être appliquée sur la peau sous forme d'aérosol. Elle peut également se présenter sous forme solide, et par exemple sous forme de stick.

Avantageusement, la composition selon l'invention est sous forme d'une émulsion huile dans eau ou d'un gel, une crème ou un sérum.

De façon connue, la composition de l'invention peut contenir également les adjuvants habituels dans les domaines cosmétique et dermatologique, tels que les gélifiants hydrophiles ou lipophiles, les actifs hydrophiles ou lipophiles, les agents hydratants (tels que la glycérine, le propylène glycol, le butylène glycol, le pentylène glycol, l'hexylène glycol, le dipropylène glycol, le diéthylène glycol), les conservateurs, les antioxydants, les solvants, les parfums, les charges, les pigments, les filtres hydrophiles, les absorbeurs d'odeur et les matières colorantes. Les quantités de ces différents adjuvants sont celles classiquement utilisées dans les domaines considérés, et par exemple de 0,01 à 20 % du poids total de la composition. Ces adjuvants, selon leur nature, peuvent être introduits dans la phase grasse, dans la phase aqueuse, dans les vésicules lipidiques et/ou dans les nanoparticules.

Lorsque la composition selon l'invention est une émulsion, la proportion de la phase grasse peut aller de 5 à 50 % en poids, et de préférence de 5 à 30 % en poids par rapport au poids total de la composition.

Comme matières grasses utilisables dans l'invention, on peut utiliser les huiles minérales, les huiles d'origine animale, les huiles de synthèse, les huiles siliconées et les huiles fluorées. On peut aussi utiliser comme matières grasses des acides gras, des cires et des gommes et en particulier les gommes de silicone.

Les émulsionnants et les coémulsionnants éventuellement utilisés dans la composition sous forme d'émulsion sont choisis parmi ceux classiquement utilisés dans le domaine considéré. Ces émulsionnants et coémulsionnants sont de préférence présents, dans la composition, en une proportion allant de 0,3 à 20 % en poids, et de préférence de 0,5 à 5 % en poids par rapport au poids total de la composition. Comme émulsionnants et coémulsionnants utilisables dans l'invention, il est particulièrement avantageux d'utiliser les esters d'acide gras et de polyol tels que le stéarate de PEG-100, le stéarate de PEG-50 et le stéarate de PEG-40, le tristéarate de sorbitane, les stéarates de sorbitane oxyéthylénés disponibles sous les dénominations commerciales Tween 20 ou Tween 60, par exemple et leurs mélanges.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras et la silice hydrophobe.

Bien entendu, l'homme de l'art veillera à choisir le ou les éventuels composés à ajouter aux compositions selon l'invention, ainsi que leur concentration, de manière telle que les propriétés avantageuses attachées intrinsèquement aux compositions conformes à l'invention ne soient pas, ou substantiellement pas, altérées par l'addition envisagée.

La présente invention concerne également un procédé de soin de la peau comprenant au moins une étape d'application sur la peau d'une composition conforme à l'invention.

Les compositions selon l'invention sont utiles pour améliorer l'effet barrière de la peau, son l'élasticité et sa fermeté. Elles sont également utiles pour densifier le derme et stimuler la synthèse de collagène de la peau.

L'invention sera maintenant illustrée à l'aide des exemples non limitatifs suivants.

### Exemple 1 : Détermination des conditions non cytotoxiques

### a) Culture des neurones sensitifs

Les neurones sensitifs sont issus de ganglions rachidiens embryonnaires de 15 jours. Les ganglions rachidiens isolés ont été placés dans du milieu froid Leibovitz (L15, PanBiotech; ref P04-27055) contenant 2% de pénicilline 10000U/mL et Streptomycine 10mg/mL (PS, PanBiotech; ref P06-07100) et 1% de BSA (BSA, Sigma Aldrich; ref P06-1391100). Les ganglions ont été dissociés par trypsination pendant 20 minutes à 37°C (Trypsin EDTA 1X, PanBiotech; ref P10-023100). La réaction a été stoppée par addition de Dulbecco's modified Eagle's medium (DMEM, PanBiotech; ref P04-03600) contenant de la DNAse I grade II (0.1 mg/ml, PanBiotech; ref P60-37780100) et 10% de sérum de veau foetal (SVF, Invitrogen; ref 10270106). Les cellules ont été dissociées mécaniquement par 3 passages dans une pipette 10mL. Les cellules ont été centrifugées à 515g pendant 10 minutes à 4°C. Le surnageant a été éliminé et le culot cellulaire a été remis en solution dans un milieu DMEM F-12 (PanBiotech; ref P04- 41450) contenant le supplément N2 (Fisher Scientific; ref 17502048), de la L-glutamine (PanBiotech; ref P0480100), 2% de PS, 10ng/mL de Nerve Growth Factor (NGF, Sigma Aldrich; ref N1408) et 5ng/mL de Neurotrophine 3 (NT3, PanBiotech; ref CB-1125032). La viabilité des cellules est établie par comptage cellulaire au bleu de trypan.

Les cellules sont ensuite ensemencées en plaques de culture à raison de 15 000 cellules par puits en plaques de 96 puits recouvertes de poly L-lysine (Greiner; ref 655936) en milieu DMEM F-12 (PanBiotech; ref P04- 41450) contenant le supplément N2 (Fisher Scientific; ref 17502048), de la L-glutamine (PanBiotech; ref P0480100), 2% de PS, 10ng/mL de Nerve Growth Factor (NGF, Sigma Aldrich; ref N1408) et 5ng/mL de Neurotrophine 3 (NT3, PanBiotech; ref CB-1125032) à 37°C et 5% de CO2.

Après 8 jours de culture, les neurones représentent 5% des cellules, les autres cellules sont constituées de cellules de Schawnn non myélinisantes et de fibroblastes.

### b) détermination des conditions non toxiques

Après 10 jours de culture, le milieu est remplacé par du milieu sans facteur de croissance contenant les compositions S1 à S3 à raison de 6 puits par condition expérimentale.

La composition S3 contient ainsi 1.6 grammes d'une solution vendue sous le nom de Lipoid, 1.333 grammes d'une solution vendue sous le nom de Proteasyl, 1.6 grammes d'une solution vendue sous le nom de Dermcom et 0.8 grammes d'une solution vendue sous le nom de Sphingokine NP dans 14,667 ml de PBS (PanBiotech, ref: P04-36500). Les compositions S2 et S1 sont des dilutions dans du PBS de la composition S3 de facteurs 5 et 10 respectivement.

Après 48 h d'incubation, le milieu de culture est éliminé et les cellules sont lavées deux fois par du PBS. Les cellules sont ensuite fixées par une solution d'éthanol-acide acétique 95-5 pendant 5 minutes à -20°C. Les cellules sont perméabilisées et les sites non spécifiques bloqués par une solution de PBS contenant 0,1% de saponine (Sigma Aldrich, ref S7900) et 1% de sérum de veau foetal pendant 15 minutes à température ambiante. Les cellules sont ensuite incubées 2h avec un anticorps monoclonal de souris anti β-tubuline (SIGMA-Aldrich, ref T8660) à la dilution de 1/400è en PBS contenant 1% de SVF et 0.1% de saponine. Cet anticorps marque les neurones sensitifs et leurs prolongements. Cet anticorps est ensuite révélé par un anticorps Alexa Fluor 488 chèvre anti-souris (Molecular probe, ref A11001) pendant 1 heure. Les noyaux cellulaires sont révélés par un marqueur fluorescent (Hoechst solution, SIGMA-Aldrich, ref B1155)

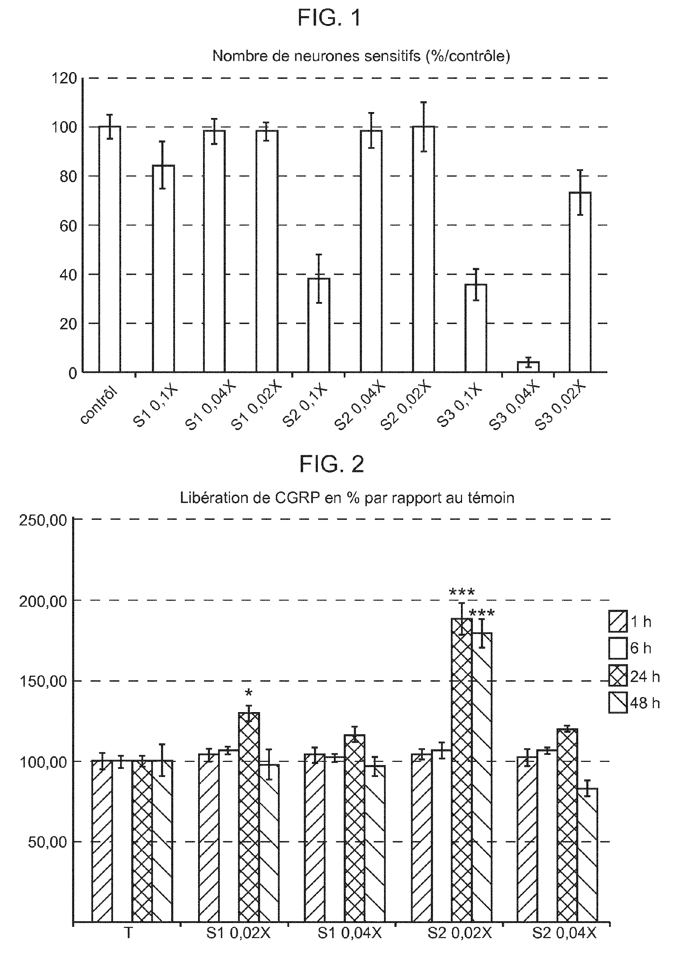

La figure 1 montre l'effet des compositions S1 à S3 à différentes dilutions allant de 0.1x à 0.04x pour chaque composition.

Comme observé sur cette figure 1, la composition S3 est toxique pour les neurones sensitifs à toutes les concentrations évaluées. Les compositions S1 et S2 sont toxiques pour les neurones sensitifs à la concentration de 0,1x mais non toxiques pour les concentrations 0,02x et 0,04x.

Les compositions S1 et S2 sont donc évaluées aux concentrations de 0,02x et 0,04x pour les expériences suivantes.

### Exemple 2 : Stimulation des neurones

Après 10 jours de culture des neurones comme dans l'exemple 1a), le milieu de culture est remplacé par du milieu de culture sans facteur de croissance contenant les compositions S1 ou S2 à raison de 12 puits par condition expérimentale.

Après 1 h, 6 h, 24 h et 48 h d'incubation, les surnageants de culture ont été récupérés et rassemblés dans deux puits par condition. Le CGRP a été dosé (n= 4) par le kit ELISA de la société Bertin selon les indications du fabriquant (ref A05482.96). Les surnageants restants ont été aliquotés en 50 µL et congelés à -80°C.

Les études statistiques ont été faites, pour la libération de CGRP par le test one way anova suivi par le test de Dunnett's multiple comparaison.

La Figure 2 montre l'effet des compositions S1 et S2 à 0.02x et 0.04x sur la libération de CGRP par les neurones sensitifs. Les données sont exprimées en pourcentage par rapport à la moyenne du témoin +/- SEM (100 % = milieu contrôle). *p<0.05, **p<0.01, ***p<0.005

Les compositions S1 et S2 à la concentration de 0.02x augmentent significativement la quantité de CGRP libérée spontanément par les neurones sensitifs en culture à partir de 24 heures d'incubation.

Le composé S2 à la concentration de 0.02x augmente également la quantité de CGRP libérée spontanément par les neurones sensitifs après 48 heures d'incubation.

Une incubation de 24 heures des neurones sensitifs en présence du composé S2 à la concentration de 0.02X semble ainsi être nécessaire pour obtenir une augmentation maximale de la quantité de CGRP libérée par les neurones sensitifs.

### Exemple 3 : Evaluation des compositions sur des explants de peau humaine in vivo

Cette étude a pour but d'évaluer l'activité de trois compositions sur les structures dermiques et épidermiques d'explants de peau humaine.

### a) Préparation des explants

24 explants d'un diamètre d'environ 12 mm chacun (±1mm) ont été préparés à partir d'une plastie abdominale d'une femme de type caucasien âgée de 46 ans (référence P1182-AB46).

Les explants ont été mis en survie en milieu BEM (BIO-EC's Explants Medium, réf Bio-EC F0120, Bio-EC Longjumeau, France) à 37°C en atmosphère humide, enrichie de 5 % de CO2.

### b) Application des produits

Les surnageants résultant de la stimulation des neurones de l'exemple 2, ont été appliqués en topique à raison de 5 µl par explant et étalés à l'aide d'une spatule à J0, J1, J2, J5, J7 et J8 selon le schéma suivant :

| **Lot** | **Traitement** | **Nb explants** | **Prélèvement** |
|---|---|---|---|
| Tp | Aucun | 3 | J-1 |
| T₀ | Aucun | 3 | J₀ |
| T | Surnageant témoin | 9 | 8h, 24h, J9 |
| P2 | Surnageant de S2 à 24h | 9 | 8h, 24h, J9 |

Les explants du lot T n'ont reçu aucun traitement excepté le renouvellement du milieu. Le milieu de culture a été renouvelé par moitié (1 mL) aux jours J1, J2, J5 et J7.

### c) Prélèvements

A J-1 et J0, chacun des 3 explants des lots Tp et T₀ a été prélevé et coupé en trois. Une partie a été fixée dans une solution de formol tamponné, une seconde partie a été congelée à -80°C et la troisième partie a été plongée dans une solution de RNA later (RNAlater® RNA stabilization Reagent, Qiagen).

A 8h, 24h et à J9, 3 explants de tous les lots ont été prélevés et traités de la même façon.

### d) Traitements histologiques

Après 24 heures de fixation dans le formol tamponné, les prélèvements ont été déshydratés et imprégnés en paraffine à l'aide d'un automate de déshydratation Leica TP 1020. Ils ont été ensuite mis en bloc à l'aide d'une station d'enrobage Leica EG 1160.

Des coupes de 5 µm ont été réalisées à l'aide d'un microtome type Minot, Leica RM 2125 et montées sur des lames de verre histologiques Superfrost®. Les observations microscopiques ont été réalisées en microscopie optique, à l'aide d'un microscope Leica type Orthoplan ou Olympus BX43. Les prises de vue ont été réalisées avec une caméra Olympus DP72 et le logiciel Cell^D.

### e) Examen de la morphologie générale

La morphologie générale a été examinée sur des coupes en paraffine colorées au trichrome de Masson variante de Goldner selon la méthode suivante :
Les coupes sur lames sont déparaffinées, réhydratée, puis soumises à un bain d'Hématoxyline de Groat et rincées à l'eau courante. Elles sont ensuite placées dans un bain de fushine-Ponceau, rincées à l'eau acétique, passées dans un bain d'Orangé G molybdique, rincées à l'eau acétique 1%, placées dans un bain Vert lumière, rincées à l'eau acétique 1%, puis déshydratées et montées en Eukitt (SIGMA-Aldrich, France).

Grâce à cette coloration, les corps cellulaires apparaissent en rose/rouge, les noyaux en violet/noir, le collagène en vert et les fibres élastiques en marron/violet.

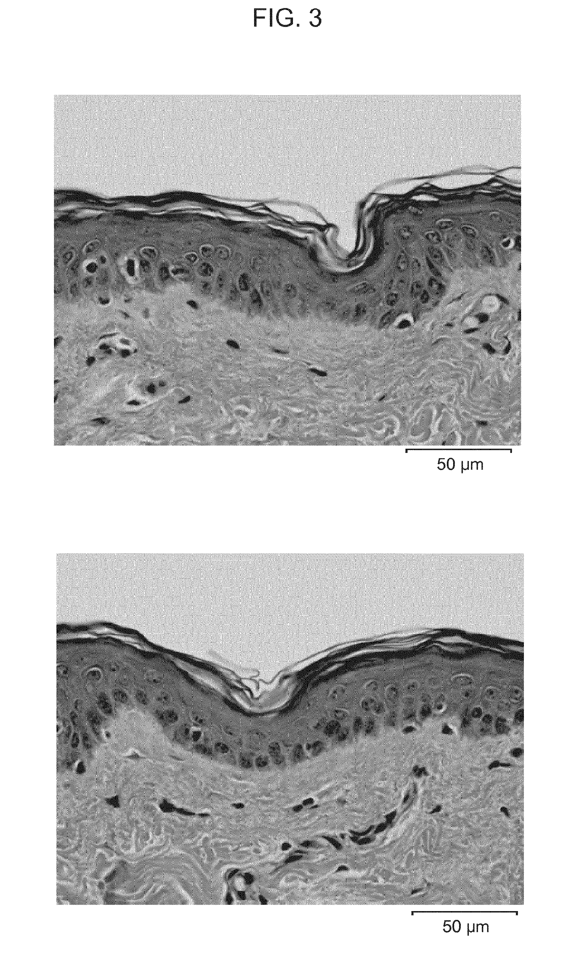

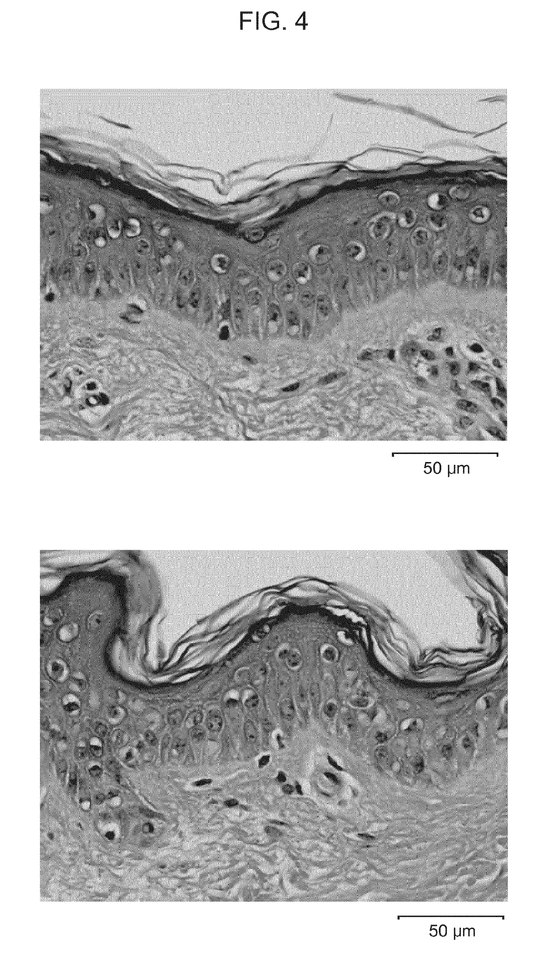

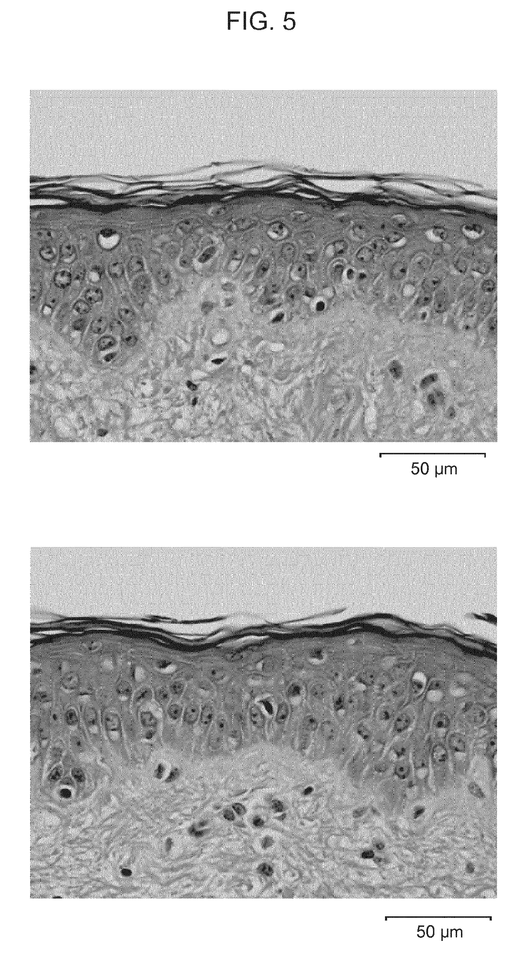

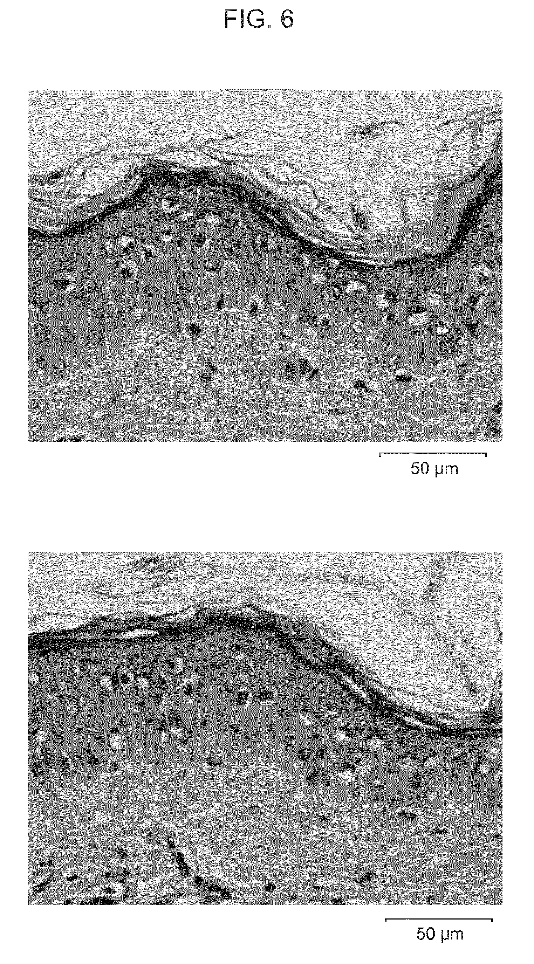

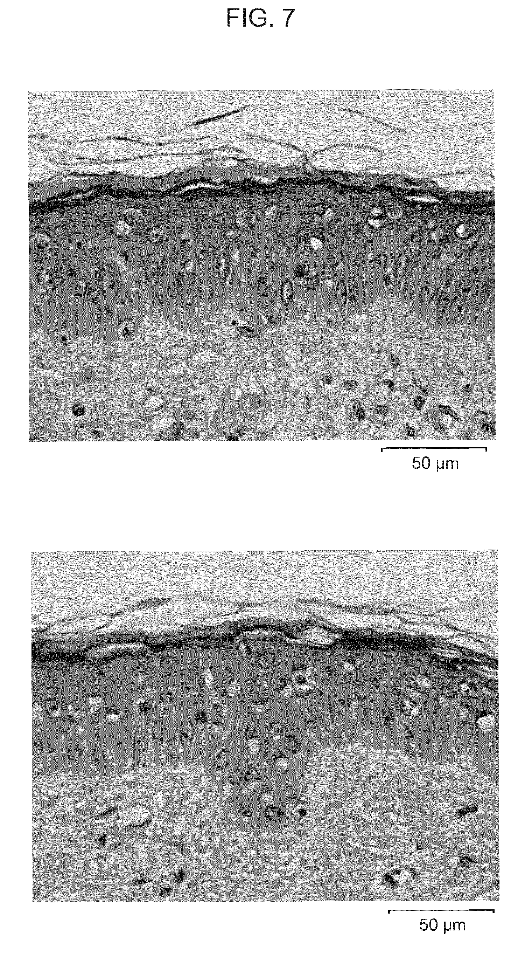

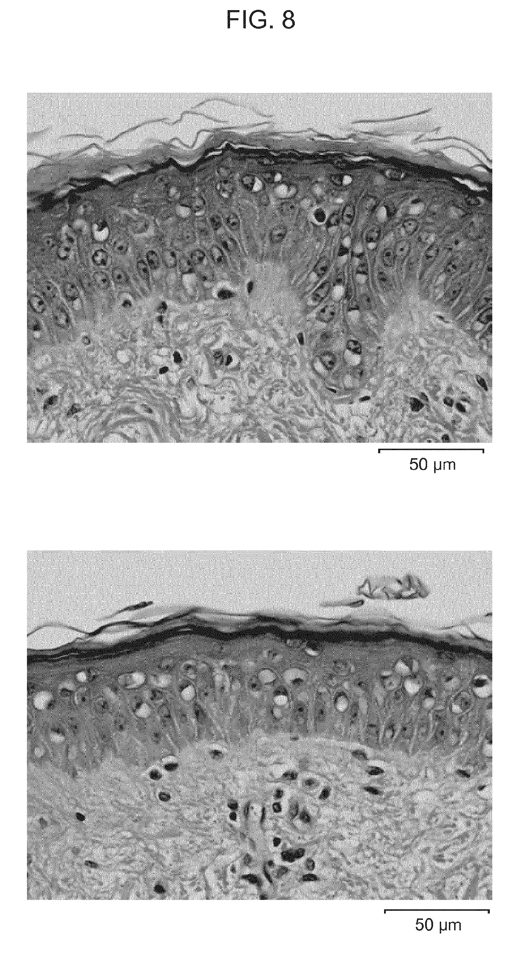

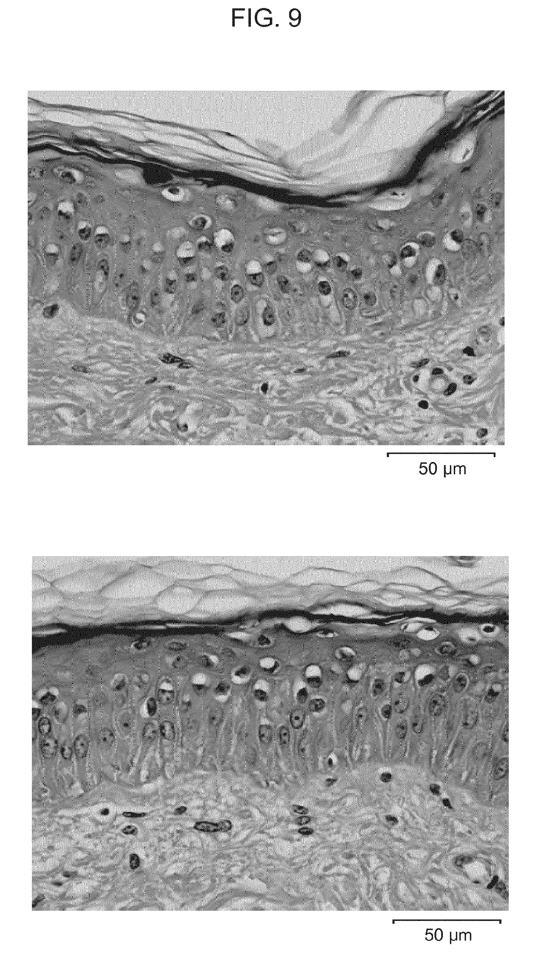

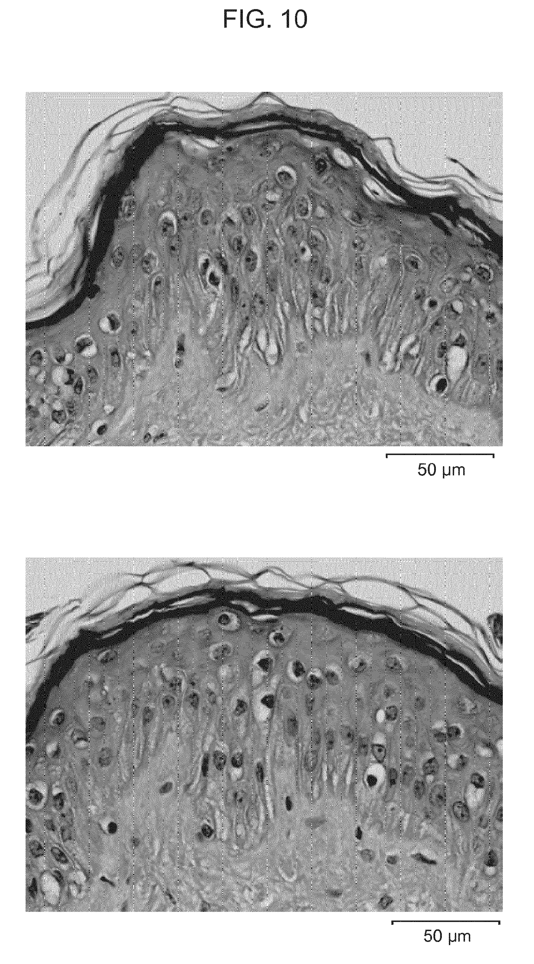

Les résultats sont les suivants:
Lot témoin plastie à J-1, (Tp) : Le *stratum corneum* est légèrement épais, légèrement feuilleté, nettement kératinisé en surface et à sa base. L'épiderme présente 3 à 4 assises cellulaires avec une bonne morphologie. Le relief de la jonction dermo-épidermique est faible. Le derme papillaire montre des faisceaux de collagène épais formant un réseau dense. La morphologie des cellules dermiques est bonne (Figure 3).
Lot témoin à J0, (T0) : Le *stratum corneum* est modérément épais, modérément feuilleté, modérément kératinisé en surface et nettement à sa base. L'épiderme présente 3 à 4 assises cellulaires avec une bonne morphologie. Le relief de la jonction dermo-épidermique est faible. Le derme papillaire montre des faisceaux de collagène épais formant un réseau assez dense. La morphologie des cellules dermiques est bonne (Figure 4).
Lot non traité à 8h, (T8h) : Le *stratum corneum* est modérément épais, légèrement feuilleté, modérément kératinisé en surface et nettement à sa base. L'épiderme présente 3 à 4 assises cellulaires avec une bonne morphologie. Le relief de la jonction dermo-épidermique est faible. Le derme papillaire montre des faisceaux de collagène épais formant un réseau modérément dense. La morphologie des cellules dermiques est bonne (Figure 5).
Lot traité avec P à 8h, (P8h) : Le *stratum corneum* est assez épais, assez bien feuilleté, légèrement kératinisé en surface et nettement à sa base. L'épiderme présente 3 à 4 assises cellulaires avec une bonne morphologie. Le relief de la jonction dermo-épidermique est faible. Le derme papillaire montre des faisceaux de collagène épais formant un réseau assez dense. La morphologie des cellules dermiques est bonne (Figure 6).
Lot non traité à 24h, (T24h) : Le *stratum corneum* est assez épais, modérément feuilleté, légèrement kératinisé en surface et nettement à sa base. L'épiderme présente 3 à 4 assises cellulaires avec une bonne morphologie. Le relief de la jonction dermo-épidermique est faible. Le derme papillaire montre des faisceaux de collagène épais formant un réseau modérément dense. La morphologie des cellules dermiques est bonne (Figure 7).
Lot traité avec P à 24h, (P24h) : Le *stratum corneum* est modérément épais, modérément feuilleté, légèrement kératinisé en surface et nettement à sa base. L'épiderme présente 3 à 4 assises cellulaires avec une bonne morphologie. Le relief de la jonction dermo-épidermique est faible. Le derme papillaire montre des faisceaux de collagène épais formant un réseau modérément dense. La morphologie des cellules dermiques est bonne (Figure 8).
Lot non traité à J9, (TJ9) : Le *stratum corneum* est assez épais, modérément feuilleté, légèrement kératinisé en surface et nettement à sa base avec une légère parakératose. L'épiderme présente 4 à 5 assises cellulaires avec une bonne morphologie. Le relief de la jonction dermo-épidermique est faible. Le derme papillaire montre des faisceaux de collagène assez épais formant un réseau modérément dense. La morphologie des cellules dermiques est bonne (Figure 9).
Lot traité avec P à J9, (PJ9) : Le *stratum corneum* est assez épais, modérément feuilleté, légèrement kératinisé en surface et nettement à sa base avec une parakeratose modérée. L'épiderme présente 4 à 5 assises cellulaires avec une légère acanthose hypertrophique et une morphologie assez bonne. Une légère spongiose est observée dans la couche basale. Le relief de la jonction dermo-épidermique est faible. Le derme papillaire montre des faisceaux de collagène épais formant un réseau dense. La morphologie des cellules dermiques est bonne (Figure 10).

Selon les conditions opératoires décrites précédemment, et comparé au lot témoin à 8h (T8h), 24h (T24h) et à J9 (TJ9), on peut déduire de ces expériences le tableau récapitulatif suivant :

| *vs* T | | Morphologie générale |
|---|---|---|
| P1 | 8h | ↗ densité du collagène dans le DP |
| | 24h | ↔ |
| | J9 | ↗ épaisseur de l'épiderme |
| | | ↗↗ densité du collagène dans le DP |

| Diminution | | Augmentation | | | |
|---|---|---|---|---|---|
| ↘ | Légère | ↗ | | ↔ | Pas de variation |
| ↘↘ | Modérée | ↗↗ | | ns | non significatif |
| ↘↘↘ | Assez Nette | ↗↗↗ | | EpiD | Epiderme |
| ↘↘↘↘ | Nette | ↗↗↗↗ | | DP | Derme papillaire |
| ↘↘↘↘↘ | Très Nette | ↗↗↗↗↗ | | | |

Le produit P présente une légère activité dermique à 8h, en augmentant la densité du collagène du derme papillaire. A J9, son activité dermique devient plus importante, en induisant une augmentation modérée de la densité du collagène du derme papillaire. De plus, il présente une légère activité épidermique, en induisant une légère augmentation de l'épaisseur de l'épiderme via une augmentation de la taille des kératinocytes.

### Exemple 4 : Composition crème soyeuse regalbante

Toutes les quantités sont indiquées en pourcentage massique de matière active par rapport au poids total de la composition.

| **Composé** | % |
|---|---|
| Eau | QSP 100 |
| Glycérine | 5,0 |
| Polyacrylate de sodium | 0,5 |
| Cellulose microcrystalline | 0,5 |
| Stéarate de glycéryl / candelilla / jojoba / esters de polyglycéryl de son de riz / lactylate stéaroyl sodium / alcool cétéarylique | 4,0 |
| Polystéarate de sucrose / polyisobutème hydrogéné | 2,5 |
| Triglycérides en C10-C18 | 2,0 |
| Acide palmitique / acide stéarique | 1,5 |
| Huile de *Cocos nucifera* | 2,0 |
| Isodécyle néopentanoate | 4,0 |
| Coco-caprylate | 3,0 |
| Diméthicone / copolymères de diméthicone | 4,0 |
| Extrait de bulbe de *Crocus chrysanthus* | 0,5 |
| Alcool / glycérine / phosphate d'ascorbyle magnésium | 0,5 |
| Extrait de *Pisum sativum* | 3,0 |
| Caprooyl phytosphingosine | 0,2 |
| Octénylsuccinate aluminium starch | 4,0 |
| Conservateurs | QS |

### Exemple 5 : Sérum liftant intensif

Toutes les quantités sont indiquées en pourcentage massique de matière active par rapport au poids total de la composition.

| **Composé** | % |
|---|---|
| Eau | QSP 100 |
| Polyacrylate de sodium | 0,8 |
| Diméthicone | 3,0 |
| Diméthicone / copolymères de diméthicone | 1,5 |
| Caprooyl phytosphingosine | 0,2 |
| Extrait de bulbe de *Crocus chrysanthus* | 0,5 |
| Alcool / glycérine / phosphate d'ascorbyle magnésium | 0,5 |
| Extrait de *Pisum sativum* | 5,0 |
| Glycérine | 5,0 |
| Fluorphlogopite synthétique / silice | 1,0 |
| Conservateurs | QS |

### Exemple 6 : Baume à lèvres repulpant

Toutes les quantités sont indiquées en pourcentage massique de matière active par rapport au poids total de la composition.

| **Composé** | % |
|---|---|
| Eau | QSP 100 |
| Glycérine | 5,00 |
| Polyacrylate de sodium | 0,70 |
| Gomme de xanthane | 0,10 |
| Cellulose microcrystalline | 0,50 |
| Stéareth-21 | 2,00 |
| Stéareth-2 | 3,00 |
| Acide palmitique / acide stéarique | 3,00 |
| Cire de fleur de *Acacia farnesiana* | 1,00 |
| Cire d'abeille | 1,00 |
| Beurre de Karité | 5,00 |
| Huile de *Cocos naucifera* | 3,00 |
| Caprooyl phytosphingosine | 0,20 |
| Coco-caprylate | 3,00 |
| Diméthicone / copolymères de diméthicone | 2,00 |
| Tocophérol | 0,03 |
| Extrait de bulbe de *Crocus chrysanthus* | 0,50 |
| Alcool / glycérine / phosphate d'ascorbyle magnésium | 0,50 |
| Extrait de *Pisum sativum* | 5,00 |
| Octénylsuccinate aluminium starch | 4,00 |
| Pentaisononanoate de dipentaerythrityl | 2,00 |
| Conservateurs | QS |

## Revendications

1. Composition comprenant dans un milieu physiologiquement acceptable:
- du phosphate d'ascorbyle de magnésium
- un extrait de *Pisum sativum*
- en extrait de bulbe de *Crocus chrysanthus*
- un composé céramide de formule (I)

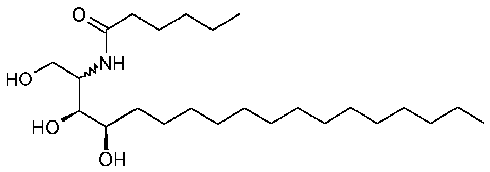

2. Composition selon la revendication 1, dans laquelle le phosphate d'ascorbyle de magnésium est présent dans des proportions de 0.1 à 10% en poids par rapport au poids total de la composition.

3. Composition selon l'une des revendications 1 à 2, dans laquelle le phosphate d'ascorbyle de magnésium est présent dans des proportions de 0.6 à 5 % en poids par rapport au poids total de la composition.

4. Composition selon l'une quelconque des revendications 1 à 3, dans laquelle l'extrait de *Pisum sativum* est présent dans des proportions de 0.2 à 10% en poids par rapport au poids total de la composition.

5. Composition selon l'une quelconque des revendications 1 à 4, dans laquelle l'extrait de *Pisum sativum* est présent dans des proportions de 0.5 à 5% en poids par rapport au poids total de la composition.

6. Composition selon l'une quelconque des revendications 1 à 5, dans laquelle l'extrait de bulbe de *Crocus chrysanthus* est présent dans des proportions de 0.2 à 10% en poids par rapport au poids total de la composition.

7. Composition selon l'une quelconque des revendications 1 à 6, dans laquelle l'extrait de bulbe de *Crocus chrysanthus* est présent dans des proportions de 0.5 à 5% en poids par rapport au poids total de la composition.

8. Composition selon l'une quelconque des revendications 1 à 7, dans laquelle le composé de formule (I) est présent dans des proportions de 0.1 à 5% en poids par rapport au poids total de la composition.

9. Composition selon l'une quelconque des revendications 1 à 8, dans laquelle le composé de formule (I) est présent dans des proportions de 0.2 à 3% en poids par rapport au poids total de la composition.

10. Composition selon l'une quelconque des revendications 1 à 9, **caractérisée par le fait qu'**elle se présente sous la forme d'émulsion huile-dans-eau, de gel, de crème, ou sous la forme d'un sérum.

11. Procédé de soin de la peau comprenant au moins une étape d'application sur la peau d'une composition telle que décrite dans l'une des revendications 1 à 10.

12. Utilisation de la composition selon l'une des revendications 1 à 10, pour améliorer l'effet barrière de la peau.

13. Utilisation de la composition selon l'une des revendications 1 à 10, pour améliorer l'élasticité et la fermeté de la peau

14. Utilisation de la composition selon l'une des revendications 1 à 10, pour densifier le derme

15. Utilisation de la composition selon l'une des revendications 1 à 10, pour stimuler la synthèse de collagène de la peau.
